# EUROPEAN PATENT SPECIFICATION

(11) **EP 0 662 967 B1**
(45) Date of publication and mention of the grant of the patent: **14.01.1998**
(21) Application number: 93921840.0
(22) Date of filing: 23.09.1993
(51) Int. Cl.: C07D 309/28

(54) **SYNTHESIS OF N-ACETYL NEURAMINIC ACID DERIVATIVES**
HERSTELLUNG VON DERIVATEN VON N-ACETYL NEURAMINICSÄURE
SYNTHESE DE DERIVES DE L'ACIDE N-ACETYLE NEURAMINIQUE

(30) Priority: 25.09.1992 GB 9220327
(43) Date of publication of application: 19.07.1995
(73) Proprietor: BIOTA SCIENTIFIC MANAGEMENT PTY. LTD., Victoria 3146 (AU)
(72) Inventor: PATEL, Vipulkumar, Glaxo Group Research Ltd., Middlesex UB6 0HE (GB)
(74) Representative: Woodman, Derek
(86) International application number: EP9302575
(87) International publication number: WO9407886

(56) References cited:
- EP-A- 0 539 204
- WO-A-91/16320
- WO-A-93/12105
- LIEBIG's ANNALEN DER CHEMIE, vol. 1991, no. 2, February 1991, Weinheim (DE); E. SCHREINER et al., pp. 129-134

## Description

The present invention relates to a process for the preparation of derivatives of N-acetyl neuraminic acid. More particularly the invention relates to a process for the preparation of 5-acetamido-2,3,4,5-tetradeoxy-4-guanidino-D-glycero-D-galactonon-2-enopyranosonic acid (the 4-guanidino analogue of DANA; also known as 5-(acetylamino)-2,6 anhydro-3,4,5-trideoxy-4-guanidino-D-glycero-D-galacto-non-2-enonic acid) and derivatives thereof.

PCT/AU91/00161 (publication no. WO91/16320) describes a number of derivatives of 5-acetamido-2,3,5-trideoxy-D-glycero-D-galacto-non-2-enopyranosonic acid (2,3-dideoxy-2,3-didehydro-N-acetyl-neuraminic acid; DANA) including the 4-guanidino analogue of DANA. The 4-guanidino analogue of DANA is prepared by the reaction of the corresponding O-acyl protected 4-amino analogue of DANA by reaction with S-methylisourea followed by deprotection.

We have now found a method of preparing the 4-guanidino analogue of DANA directly from the unprotected 4-amino analogue of DANA.

The structure of the 4-amino and 4-guanidino analogues of DANA are shown below:

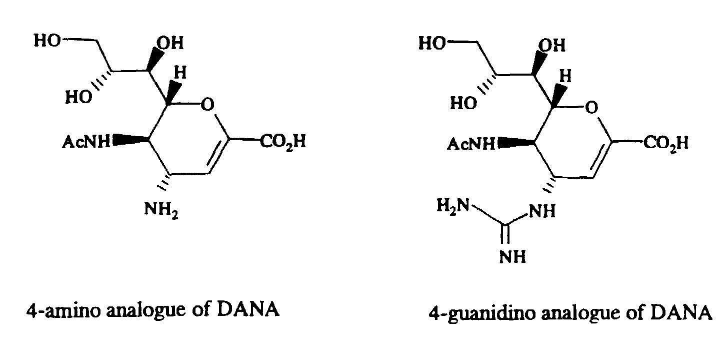

The invention thus provides in a first aspect a method for the preparation of 5-acetamido-2,3,4,5-tetradeoxy-4-guanidino-D-glycero-D-galacto-non-2-enopyranosonic acid which comprises the reaction of 5-acetamido-4-amino-2,3,4,5-tetradeoxy D-glycero-D-galacto-non-2-enopyranosonic acid with a compound of formula (I) 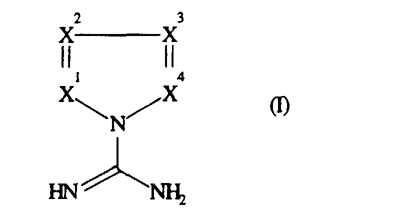 where at least one of X¹, X², X³ and X⁴ is C-R and the remainder are C-R or N and each R is the same or different and is H, C₁₋₆ alkyl, unsubstituted or substituted phenyl, unsubstituted or substituted phenyl C₁₋₄ alkyl or an electron withdrawing group.

When R in the compound of formula (I) is an electron withdrawing group any such group may be employed. Such groups will be evident to those skilled in the art and include for example carboxyl, nitro and cyano.

The compound of formula (I) may be employed either as the free base or as an acid addition salt. Suitable salts include those derived from inorganic or organic acids such as hydrochloric, hydrobromic, sulphuric, nitric, perchloric, fumaric, maleic, phosphoric, glycollic, lactic, salicylic, succinic, toluene- p-sulphonic, tartaric, acetic, citric, methanesulphonic, formic, benzoic, malonic, naphthalene-2-sulphonic and benzenesulphonic acids.

The preferred compounds of formula (I) are pyrazole-1H-carboxamidine and derivatives thereof bearing a C₁₋₆ alkyl group on the pyrazole ring.

A particularly preferred compound of formula (I) is pyrazole-1H-carboxamidine either as the free base or, preferably, as an acid addition salt such as pyrazole-1H-carboxamidine hydrochloride (PCH).

The reaction is conveniently carried out in aqueous medium. By aqueous medium is meant any liquid medium comprising a substantial amount, for example 50% or more, of water. Preferably the aqueous medium comprises water alone.

The reaction may be carried at ambient or elevated temperature for example 30°C to 70°C. Preferably the reaction is carried out at about 50-55°C.

The molar ratio of the 4-amino analogue of DANA to the compound of formula (I) employed in the reaction may be from about 1:1 to about 1:10 for example 1:1 to 1:3. Preferably the compound of formula (I) is employed in a molar excess of about 1.5 - 2 fold, e.g. about 1.8 fold.

The reaction is carried out at a pH range of about 6 to about 9. The pH range may vary within this range during the reaction.

Where the compound of formula (I) is employed in the form of an acid addition salt the pH may be maintained in the desired range by addition of one or more inorganic or organic bases. Such bases include for example alkali metal hydroxides and carbonates such as lithium hydroxide, sodium carbonate or sodium bicarbonate and amines such as triethylamine, imidazole or 1,8-diazabicylo [5.4.0]undec-7-ene (DBU). The amount of base required to maintain the desired pH will depend upon the particular base(s) employed. Quantities of the base(s) required to control the pH will be apparent to those skilled in the art.

The desired 4-guanidino analogue of DANA may be isolated by any conventional method from the reaction mixture, for example by crystallisation or chromatography. In particular the 4-guanidino analogue of DANA may be isolated by treatment of an aqueous solution with a water miscible organic solvent in which the compound is insoluble. Such solvents include for example aliphatic alcohols such as methanol and isopropyl alcohol (IPA) and acetone.

The present invention is further described by the following examples which are for illustrative purposes only and should not be construed as a limitation of the invention.

### Example 1

### 5-Acetamido-2,3,4,5-tetradeoxy-4-guanidino-D-glycero-D-galacto-non-2-enopyranosonic acid

To a stirred solution of 5-acetamido-4-amino-2,3,4,5-tetradeoxy-D-glycero-D-galacto-non-2-enopyranosonic acid as the trihydrate (58.05g; 169mmol) in water (300ml) at 33°C was added in one lot pyrazole-1H-carboxamidine hydrochloride (600mmol, 87.95g) followed by triethylamine (60ml, pH = 8.4) and the reaction stirred for 5 hrs.

The crude reaction mixture was added to rapidly stirred methanol (900ml). Stirring was continued overnight and the precipitated solid was collected, washed with 4:1 MeOH: H₂O (250ml), air dried and dried in a vacuum oven (42°C) for 2 h. Yield = 46.7g.

The so obtained product (45.5g) was suspended in water (518ml) and heated with rapid stirring. The so obtained solution was rapidly filtered and allowed to cool to ambient temperature. The solution was further cooled (ice-water bath) to 3°C. *Iso*propyl alcohol (450ml) was added dropwise to the cold solution over 1 hr and stirring continued over 1.5hr. The precipitated solid was filtered off, and dried at 40°C to give the title compound (30.4g) identical with authentic material.

### Example 2

### 5-Acetamido-2,3,4,5-tetradeoxy-4-guanidino-D-glycero- D-galacto-non-2-enopyranosonic acid

A mixture of 5-acetamido-4-amino-2,3,4,5-tetradeoxy-D-glycero-D-galacto-non-2-enopyranosonic acid as the trihydrate (15.0g), pyrazole-1H-carboxamidine hydrochloride (7.55g) and imidazole (11.55g) in water (52.5ml) was stirred and heated at 50-55°. After 18h the resulting slurry was treated with acetone (150ml) over 15 min. maintaining the vesel contents at 50-55°. The mixture was then cooled to 15-20° and after a further 3h the product was collected by vacuum filtration. The bed was washed with 4:1 acetone/water (2x25ml) and then acetone (25ml). The product was air dried at ambient temperature to give the title compound (10.98g).
- PMR(D₂O): 2.04 (3H, s), 3.67 (2H, m), 3.93 (2H, m), 4.23 (1H,m), 4.42 (2H,m) 5.63 (1H, d, J 2.5Hz).
- IR (Nujol): 3428, 3338, 3253; NH, OH
1692, 1666, 1646, 1619, 1575; CO (CH₃CONH, CO₂⁻), CN.

## Claims

1. A method for the preparation of 5-acetamido-2,3,4,5-tetradeoxy-4-guanidino-D-glycero-D-galacto-non-2-eno-pyranosonic acid which comprises the reaction of 5-acetamido-4-amino-2,3,4,5-tetradeoxy-D-glycero-D-galacto-non-2-enopyranosonic acid with a compound of formula (I) 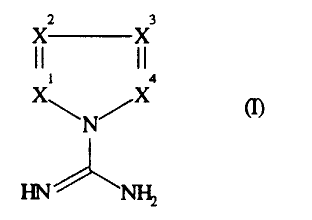
where at least one of X¹, X², X³ and X⁴ is C-R and the remainder are C-R or N
and each R is the same or different and is H, C₁₋₆ alkyl, unsubstituted or substituted phenyl, unsubstituted or substituted phenyl C₁₋₄ alkyl or an electron withdrawing group.

2. A method as claimed in claim 1 wherein the compound of formula (I) is pyrazole-1H-carboxamidine or a derivative thereof bearing a C₁₋₆ alkyl group on the pyrazole ring.

3. A method as claimed in claim 2 wherein the compound of formula (I) is pyrazole-1H-carboxamidine.

4. A method as claimed in any one of claims 1 to 3 wherein the compound of formula (I) is in the form of an acid addition salt.

5. A method as claimed in any one of claims 1 to 4 wherein the salt is the hydrochloride salt.

6. A method as claimed in any one of claims 1 to 5 wherein the reaction is carried out in an aqueous medium.

7. A method as claimed in claim 6 wherein the aqueous medium is water.

8. A method as claimed in any one of claims 1 to 7 wherein the process is carried out at a temperature of 30-70°C.

9. A method as claimed in claim 8 wherein the temperature is from 50-55°C.

10. A method as claimed in any one of claims 1 to 9 wherein the molar ratio of 5-acetamido-2,3,4,5-tetradeoxy-4-amino-D-glycero-D-galacto-non-2-enopyranosonic acid to the compound of formula (I) is in the range of 1:1 to 1:10.

11. A method as claimed in claim 10 wherein the ratio is about 1: 1.8.

12. A method as claimed in any one of claims 1 to 11 wherein the pH is in the range of 6-9.

## Patentansprüche

1. Verfahren zur Herstellung von 5-Acetamido-2,3,4,5-tetradesoxy-4-guanidino-D-glycero-D-galacto-non-2-eno-pyranosonsäure, umfassend die Reaktion von 5-Acetamido-4-amino-2,3,4,5-tetradesoxy-D-glycero-D-galacto-non-2-eno-pyranosonsäure mit einer Verbindung der Formel (I) 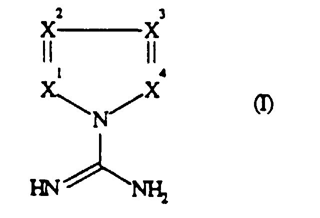
worin mindestens eine der Gruppen X¹, X², X³ und X⁴ C-R darstellt und die übrigen Gruppen davon C-R oder N darstellen
und jeder der Reste R gleich oder verschieden ist und H, C₁₋₆-Alkyl, unsubstituiertes oder substituiertes Phenyl, unsubstituiertes oder substituiertes Phenyl-C₁₋₄-alkyl oder eine elektronenziehende Gruppe darstellt.

2. Verfahren nach Anspruch 1, wobei die Verbindung der Formel (I) Pyrazol-1H-carboxamidin oder ein Derivat davon, das eine C₁₋₆-Alkylgruppe am Pyrazolring trägt, ist.

3. Verfahren nach Anspruch 2, wobei die Verbindung der Formel (I) Pyrazol-1H-carboxamidin ist.

4. Verfahren nach einem der Ansprüche 1 bis 3, wobei die Verbindung der Formel (I) in Form eines Säureadditionssalzes vorliegt.

5. Verfahren nach einem der Ansprüche 1 bis 4, wobei das Salz das Hydrochloridsalz ist.

6. Verfahren nach einem der Ansprüche 1 bis 5, wobei die Umsetzung in einem wässerigen Medium ausgeführt wird.

7. Verfahren nach Anspruch 6, wobei das wässerige Medium Wasser ist.

8. Verfahren nach einem der Ansprüche 1 bis 7, wobei das Verfahren bei einer Temperatur von 30-70°C ausgeführt wird.

9. Verfahren nach Anspruch 8, wobei die Temperatur 50-55°C ist.

10. Verfahren nach einem der Ansprüche 1 bis 9, wobei das Molverhältnis von 5-Acetamido-2,3,4,5-tetradesoxy-4-amino-D-glycero-D-galacto-non-2-eno-pyranosonsäure zu der Verbindung der Formel (I) im Bereich von 1:1 bis 1:10 liegt.

11. Verfahren nach Anspruch 10, wobei das Verhältnis etwa 1:1,8 ist.

12. Verfahren nach einem der Ansprüche 1 bis 11, wobei der pH-Wert im Bereich von 6-9 liegt.

## Revendications

1. Procédé pour la préparation de l'acide 5-acétamido-2,3,4,5-tétradésoxy-4-guanidino-D-glycéro-D-galacto-non-2-èno-pyranosonique comprenant la réaction de l'acide 5-acétamido-4-amino-2,3,4,5-tétradésoxy-D-glycéro-D-galacto-non-2-ènopyranosonique avec un composé de formule (I) 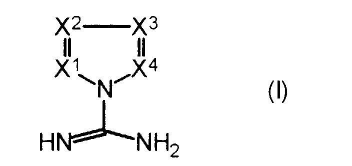
dans laquelle au moins l'un parmi X¹, X², X³ et X⁴ est C-R les autres étant C-R ou N
et chaque R, identique ou différent, est H, C₁-C₆ alkyle, phényle substitué ou non substitué, phényle-C₁-C₄ alkyle substitué ou non substitué ou un groupe électro-attracteur.

2. Procédé selon la revendication 1 dans lequel le composé de formule (I) est une pyrazole-1H-carboxamidine ou un dérivé de celle-ci portant un groupe C₁-C₆ alkyle sur le noyau pyrazole.

3. Procédé selon la revendication 2 dans lequel le composé de formule (I) est une pyrazole-1H-carboxamidine.

4. Procédé selon l'une quelconque des revendications 1 à 3 dans lequel le composé de formule (I) est sous la forme d'un sel d'addition avec un acide

5. Procédé selon l'une quelconque des revendications 1 à 4 dans lequel le sel est un sel de type chlorhydrate.

6. Procédé selon l'une quelconque des revendications 1 à 5 dans lequel la réaction est réalisée en milieu aqueux.

7. Procédé selon la revendication 6 dans lequel le milieu aqueux est l'eau.

8. Procédé selon l'une quelconque des revendications 1 à 7 dans lequel le procédé est réalisé à une température comprise entre 30 et 70°C.

9. Procédé selon la revendication 8 dans lequel la température est de 50 à 55°C.

10. Procédé selon l'une quelconque des revendications 1 à 9 dans lequel le rapport molaire de l'acide 5-acétamido-2,3,4,5-tétradésoxy-4-amino-D-glycéro-D-galacto-non-2-èno-pyranosonique au composé de formule (I) est situé dans la plage 1:1 à 1:10.

11. Procédé selon la revendication 10 dans lequel le rapport est d'environ de 1:1,8.

12. Procédé selon l'une quelconque des revendications 1 à 11 dans lequel le pH est compris entre 6 et 9.
